(19) Europäisches Patentamt / European Patent Office / Office européen des brevets

(11) **EP 4 481 376 A1**

(12) **EUROPEAN PATENT APPLICATION**

(43) Date of publication:
25.12.2024 Bulletin 2024/52

(21) Application number: 24182272.5

(22) Date of filing: 14.06.2024

(51) International Patent Classification (IPC):
*G01N 27/447* (2006.01)

(52) Cooperative Patent Classification (CPC):
**G01N 27/44769**; G01N 27/44721

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.06.2023 JP 2023102011**

(71) Applicant: **ARKRAY, Inc.**
**Kyoto-shi, Kyoto 601-8045 (JP)**

(72) Inventor: **ONUMA, Naotsugu**
**Kyoto, 602-0008 (JP)**

(74) Representative: **Dehns**
**10 Old Bailey**
**London EC4M 7NG (GB)**

(54) **SAMPLE ANALYSIS METHOD AND SAMPLE ANALYSIS APPARATUS**

(57)    Provided are a sample analysis method and a sample analysis apparatus capable of specifying a component included in a sample regardless of a concentration of the component included in the sample. A sample analysis method or the like includes a step of separating plural components included in a sample by capillary electrophoresis; a step of obtaining an electropherogram of the sample; a step of acquiring a detection time of each peak and a concentration corresponding to each peak from the electropherogram; and a step of specifying a component included in the sample using a parameter indicating a correlation between a detection time and a concentration calculated using a known component with respect to the acquired detection time and the acquired concentration.

FIG.1

EP 4 481 376 A1

**Description**

BACKGROUND

Technical Field

The present disclosure relates to a sample analysis method and a sample analysis apparatus.

Related Art

Analysis of hemoglobin (hereinafter, also referred to as "Hb") is routinely performed in the field of clinical inspection. The Hb species to be analyzed varies depending on a purpose of inspection. HbA1c is well known as an analysis target for diagnosis and disease state determination of diabetes. Mutant Hb (or "hemoglobin variants") represented by HbS (sickle cell Hb), HbC, HbD, HbE, and the like is used as an analysis target for diagnosis of abnormal Hb disease. HbA2 and HbF (fetal Hb) are widely used as analysis targets for diagnosis of β-thalassemia.

The analysis of Hb is performed by a high performance liquid chromatography (HPLC) method such as an ion exchange chromatography method, a capillary electrophoresis (CE) method, or the like.

Japanese Patent Application Laid-Open (JP-A) No. 2016-136135 describes a method of analyzing HbA2 by anion exchange electrokinetic chromatography using a solution containing a cationic polymer as a pseudo stationary phase.

Japanese Patent No. 6846324 and Japanese Patent No. 6871129 describe an analysis method of a method of identifying a component such as HbA1c using an interface reaching time point by ion exchange electrokinetic chromatography.

SUMMARY OF THE INVENTION

When the method described in JP-ANo. 2016-136135 is used, it is possible to downsize an apparatus and shorten a measurement time. By combining with the method described in Japanese Patent No. 6846324 or Japanese Patent No. 6871129, it is possible to specify the Hb species of each peak based on a relative detection time by a simple apparatus. However, in these methods, when a Hb concentration in a specimen varies, peaks may overlap. When the peaks overlap, it is difficult to specify the Hb species.

At least preferred embodiments of the invention provide a sample analysis method and a sample analysis apparatus capable of specifying a component included in a sample regardless of a concentration of the component included in the sample.

A first aspect of the present invention provides a sample analysis method including:

a step of separating a plurality of components included in a sample by capillary electrophoresis;
a step of obtaining an electropherogram of the sample;
a step of acquiring a detection time of each peak and a concentration corresponding to each peak from the electropherogram; and
a step of specifying a component included in the sample using a parameter indicating a correlation between a detection time and a concentration calculated using a known component with respect to the acquired detection time and the acquired concentration.

Optionally, the step of obtaining the electropherogram of the sample includes:

a step of optically measuring the sample to obtain an absorbance,
a step of acquiring the absorbance as an original waveform plotted on a two-dimensional plane along a time axis, and
a step of acquiring a time differential waveform that is a waveform obtained by differentiating the original waveform along the time axis,
the step of acquiring the detection time of each peak and the concentration corresponding to each peak from the electropherogram includes:

a step of acquiring a detection time of each peak in the time differential waveform and acquiring an absorbance corresponding to the acquired detection time of each peak, and
the step of specifying the component included in the sample includes:
a step of specifying a component included in the sample using a parameter indicating a correlation between a detection time and an absorbance calculated using a known component with respect to the acquired detection time and the acquired absorbance.

Optionally, the parameter indicating the correlation between the detection time and the concentration is a parameter obtained by plotting the detection time and the absorbance on the two-dimensional plane using the known component.

Optionally, the parameter indicating the correlation between the detection time and the concentration is data of a slope of an approximate straight line for each component, obtained by plotting the detection time and the absorbance on the two-dimensional plane using the known component, and

the step of specifying the component included in the sample includes:

a step of correcting the acquired detection time using the data of the slope and calculating a correction detection time, and

a step of specifying a component included in the sample based on the correction detection time.

Optionally, the parameter indicating the correlation between the detection time and the concentration is data of a boundary line separating each component, obtained by plotting the detection time and the absorbance on the two-dimensional plane using the known component, and

the step of specifying the component included in the sample includes:

a step of specifying a region to which the acquired detection time and the acquired absorbance belong, in each region separated by the boundary line using the data of the boundary line, and

a step of specifying a component included in the sample based on the specified region.

Optionally, the component is hemoglobin.

A second aspect of the present invention provides a sample analysis apparatus including:

means for separating a plurality of components included in a sample by capillary electrophoresis;

means for obtaining an electropherogram of the sample;

means for acquiring a detection time of each peak and a concentration corresponding to each peak from the electropherogram; and

means for specifying a component included in the sample using a parameter indicating a correlation between a detection time and a concentration calculated using a known component with respect to the acquired detection time and the acquired concentration.

The sample analysis apparatus of the second aspect may be configured to carry out the method of the first aspect, optionally including any of the optional features thereof.

According to an embodiment of the invention, there is provided a sample analysis method and a sample analysis apparatus capable of specifying a component included in a sample regardless of a concentration of the component included in the sample.

BRIEF DESCRIPTION OF THE DRAWINGS

Fig. 1A is a top view illustrating an embodiment of a capillary electrophoresis chip;

Fig. 1B is a cross-sectional diagram of the electrophoresis chip illustrated in Fig. 1A;

Fig. 2 is a diagram illustrating an example of an original waveform;

Fig. 3 is a diagram illustrating a time differential waveform with respect to the original waveform in Fig. 2;

Fig. 4 is a diagram in which a horizontal axis is a relative detection time PkTimeRt and a vertical axis is an absorbance TlAbs;

Fig. 5 is a diagram illustrating an example of a relationship between Hb species and a correction detection time;

Fig. 6 is a diagram in which a horizontal axis is a relative detection time PkTimeRt and a vertical axis is an absorbance TlAbs;

Fig. 7 is a system schematic diagram illustrating a sample analysis system that can be used to perform a sample analysis method of the present embodiment;

Fig. 8 is a plan diagram illustrating an analysis chip used in the sample analysis system of Fig. 7;

Fig. 9 is a cross-sectional diagram taken along line III-III of Fig. 8;

Fig. 10 is a block diagram illustrating a hardware configuration of a control unit; and

Fig. 11 is a diagram illustrating a relationship between Hb species and a relative detection time.

DETAILED DESCRIPTION OF THE INVENTION

Hereinafter, an embodiment which is an example of the present invention will be described. These descriptions and examples illustrate the embodiment and do not limit the scope of the invention.

In the numerical ranges described herein, the upper limit value or the lower limit value described in one numerical range may be replaced with the upper limit value or the lower limit value of the numerical range described elsewhere. In the numerical range described herein, the upper limit value or the lower limit value of the numerical range may be replaced with a value shown in Examples.

When referring to an amount of each component in a composition, in a case in which there are a plurality of substances corresponding to each component in the composition, it means a total amount of the plurality of substances present in the composition unless otherwise specified.

Herein, the term "step" includes not only an independent step but also a step that cannot be clearly distinguished from other steps as long as the intended purpose of the step is achieved.

Herein, a combination of two or more preferred features is a more preferred feature.

When the embodiment is described with reference to the drawings in the disclosure, the configuration of the embodiment is not limited to the configuration illustrated in the drawings. Sizes of members in each drawing are conceptual, and a relative relationship between the sizes of the members is not limited thereto.

[Sample Analysis Method]

A sample analysis method includes: a step (hereinafter, also referred to as "separation step") of separating a plurality of components included in a sample by capillary electrophoresis; a step (hereinafter, also referred to as "electropherogram acquisition step") of obtaining an electropherogram of the sample; a step (hereinafter, also referred to as "detection time and concentration acquisition step") of acquiring a detection time of each peak and a concentration corresponding to each peak from the electropherogram; and a step (hereinafter, also referred to as "component specification step") of specifying a component included in the sample using a parameter indicating a correlation between a detection time and a concentration calculated using a known component with respect to the acquired detection time and the acquired concentration.

According to the sample analysis method, the component included in the sample can be specified regardless of the concentration of the component included in the sample.

For example, a genotype of a mutant hemoglobin carrier is a heterozygote of HbA/HbV (HbV is mutant hemoglobin other than HbA) or a homozygote of HbV/HbV

Compared with blood of a person having the heterozygote, blood of a person having the homozygote has a higher concentration of Hb (HbV), and a detection time of a peak tends to be advanced. When the detection time of a peak is advanced, a plurality of peaks may overlap.

Conventionally, in the specification of Hb species included in a sample, it is sometimes difficult to specify the Hb species in a case in which the peaks overlap.

The present inventor has found that there is a correlation between a detection time of each peak and a concentration corresponding to each peak that can be acquired from an electropherogram. Further, it was found that the component included in the sample can be accurately specified regardless of the concentration of each component by using a parameter indicating a correlation between a detection time and a concentration calculated using a known component.

<Separation Step>

A sample analysis method includes a step of separating a plurality of components included in a sample by capillary electrophoresis.

The sample may be in any state such as a liquid, a solid, or a gas as long as it includes a component that can be analyzed by a capillary electrophoresis method and can be dissolved in a solvent.

Examples of the component to be analyzed among the components included in the sample include hemoglobin (Hb), albumin (Alb), globulin ($\alpha$1, $\alpha$2, $\beta$, $\gamma$globulin), fibrinogen, and the like. Examples of the hemoglobin include a plurality of hemoglobin species such as HbA, HbA2, HbF (fetal hemoglobin), or a mutant (variant) hemoglobin (HbC, HbD, HbE, HbG, HbS, or the like). Both the whole blood of the healthy person and the whole blood of the mutant hemoglobin carrier include a small amount of HbA2. These components are suitable for analysis by the capillary electrophoresis method because amino acid mutations as constituent elements are easily electrically reflected in protein molecules.

Depending on the component to be analyzed, the sample may be pretreated with an appropriate reagent or preliminarily separated by a separate method (for example, a chromatography method).

Separation of Hb is usually performed in an electrophoresis liquid. The electrophoresis liquid may be an alkaline solution or an acidic solution, but is preferably an alkaline solution.

The sample contains, for example, HbA2, HbE, HbA, HbF, HbC, HbD, HbG, HbS, and the like.

The separation of Hb in the sample by the capillary electrophoresis in the alkaline solution can be performed by using an apparatus including a capillary channel.

Specifically, it can be performed by introducing a sample into a capillary channel filled with an alkaline solution, and applying a voltage to the whole or a part of the capillary channel after the introduction of the sample. By applying the voltage, Hb in the sample can be electrophoresed and separated.

The voltage can be applied to the capillary channel by bringing a negative electrode into contact with a sample introduction side of the capillary channel and bringing a positive electrode into contact with an alkaline solution supply side.

A cross-sectional shape of the capillary channel is not particularly limited, and may be a circular shape, a rectangular shape, or other shapes.

In the case of the rectangular shape, a channel height and a channel width of the capillary channel are preferably from 1 $\mu$m to 1000 $\mu$m, more preferably from 10 $\mu$m to 200 $\mu$m, and still more preferably from 25 $\mu$m to 100 $\mu$m, respectively.

In the case of the circular shape, an inner diameter of the capillary channel is preferably 10 $\mu$m or more or 25 $\mu$m or more, and preferably 100 $\mu$m or less or 75 $\mu$m or less.

The channel length of the capillary channel is preferably from 5 mm to 150 mm, more preferably from 10 mm to 150 mm, and still more preferably from 20 mm to 60 mm.

A material of the capillary channel is not particularly limited, and examples thereof include glass, fused silica, plastic, and the like. Examples of the plastic include polymethyl methacrylate (PMMA), polycarbonate, polystyrene, polytetrafluoroethylene (PTFE), polyetheretherketone (PEEK), and the like.

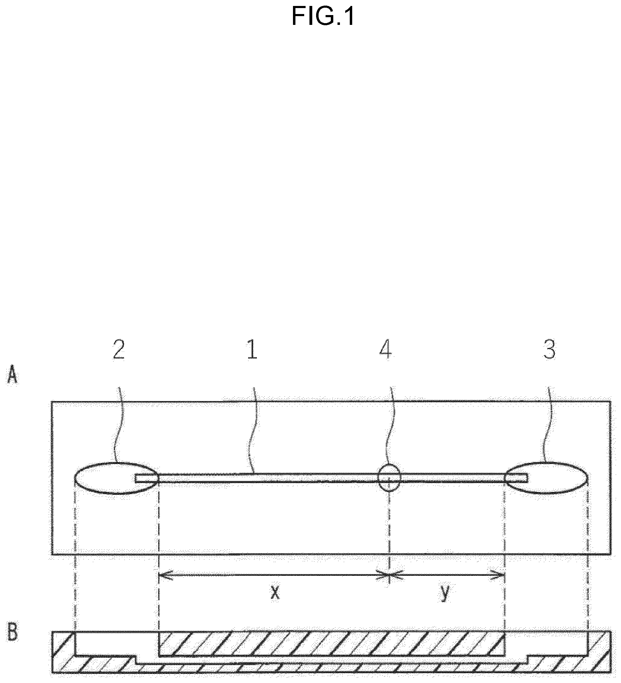

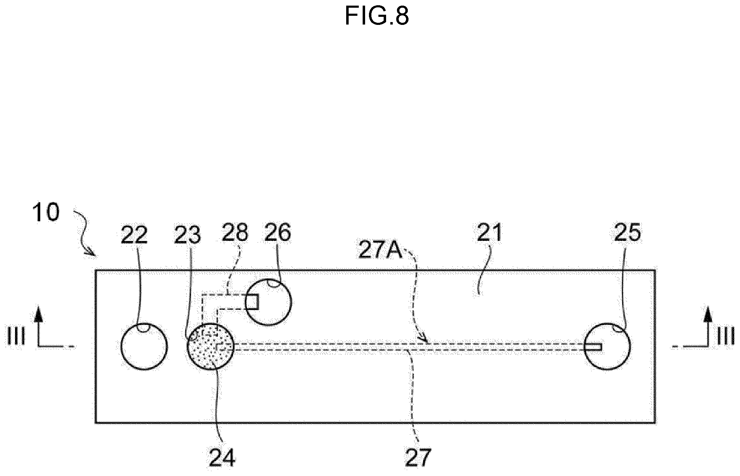

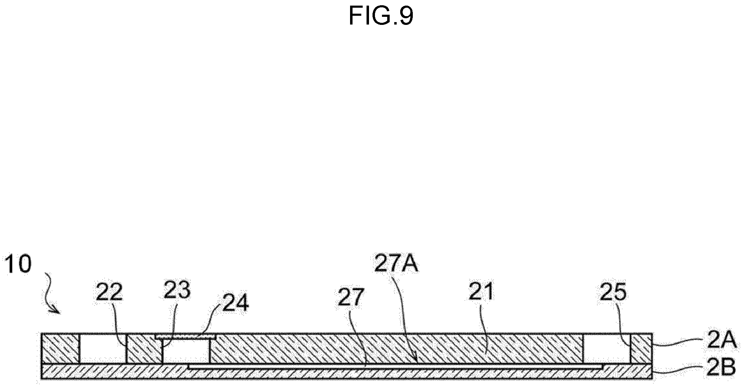

In the separation step, a capillary electrophoresis chip in which the capillary channel is made into a microchip may be used. A capillary electrophoresis chip illustrated in Figs. 1A and 1B to be described later and an analysis chip illustrated in Figs. 8 and 9 to be described later are examples of the chips used in the capillary electrophoresis method.

The capillary electrophoresis chip can have a sample holding tank, an electrophoresis liquid holding tank, and a capillary channel, and the sample holding tank and the electrophoresis liquid holding tank communicate with each other through the capillary channel.

A size of the capillary electrophoresis chip is not particularly limited, and is preferably appropriately chosen. The size of the capillary electrophoresis chip can be, for example, from 10 mm to 200 mm in length, from 1 mm to 60 mm in width, and from 0.3 mm to 5 mm in thickness.

Volumes of the sample holding tank and the electrophoresis liquid holding tank are appropriately determined according to the inner diameter and the length of the capillary channel, and are each preferably from 1 mm$^3$ to 1000 mm$^3$, and more preferably from 5 mm$^3$ to 100 mm$^3$.

An amount of the sample to be filled in the sample holding tank is not particularly limited, and may be from 1 $\mu$L to 30 $\mu$L.

An amount of the alkaline solution filled in the electrophoresis liquid holding tank is not particularly limited, and may be from 1 $\mu$L to 30 $\mu$L.

The voltage applied to both ends of the capillary channel is preferably from 500 V to 20000 V, more preferably from 500 V to 10000 V, and still more preferably from 500 V to 5000 V

In the capillary channel, a liquid flow from a negative electrode side toward a positive electrode side may be generated. Examples of the liquid flow include an electro-osmotic flow or the like.

An inner wall of the capillary channel is preferably coated with a cationic substance or an anionic substance.

By covering the inner wall of the capillary channel with the cationic substance, the inner wall of the capillary channel can be positively charged. As a result, the electro-osmotic flow from the negative electrode side toward the positive electrode side can be easily generated in the capillary channel.

When the inner wall of the capillary channel is coated with the anionic substance, the inner wall of the capillary channel is negatively charged, but the cationic polymer contained in the alkaline solution is coupled to the inner wall of the negatively charged capillary channel. As a result, the inner wall of the capillary channel is positively charged, and the electro-osmotic flow from the negative electrode side toward the positive electrode side can be easily generated in the capillary channel as described above.

The cationic substance is not particularly limited, and a silane coupling agent or the like having a cationic functional group can be used. From the viewpoint of improving a separation accuracy, the cationic substance is preferably a polymer having a quaternary ammonium base, and more preferably polydiallyldimethylammonium chloride.

The anionic substance is not particularly limited, and polysaccharides having an anionic group, silane coupling agents having an anionic functional group, and the like can be used.

Examples of the polysaccharides having an anionic group include sulfated polysaccharides, carboxylated polysaccharides, sulfonated polysaccharides, phosphorylated polysaccharides, and the like.

Examples of the sulfated polysaccharides include chondroitin sulfate, heparin, heparan, fucoidan, and salts thereof. Examples of the carboxylated polysaccharides include alginic acid, hyaluronic acid, and salts thereof.

Figs. 1A and 1B illustrate an embodiment of the capillary electrophoresis chip. Fig. 1A is a top view illustrating the embodiment of the capillary electrophoresis chip, and Fig. 1B is a cross-sectional view of the electrophoresis chip illustrated in Fig. 1A.

The capillary electrophoresis chip illustrated in Figs. 1A and 1B includes a capillary channel 1, a sample holding tank 2, and an electrophoresis liquid holding tank 3, and the sample holding tank 2 and the electrophoresis liquid holding tank 3 communicate with each other through the capillary channel 1. A detection unit 4 is formed in the capillary channel 1.

The sample holding tank 2 and the electrophoresis liquid holding tank 3 may each include an electrode for applying a voltage to both ends of the capillary channel 1 (not illustrated). Specifically, the sample holding tank 2 (sample introduction side) may include a negative electrode, and the electrophoresis liquid holding tank 3 (alkaline solution supply side) may include a positive electrode.

A position of the detection unit 4, that is, a length required for the separation (a distance from the sample holding tank 2 to the detection unit 4, x in Fig. 1A) can be appropriately determined according to the length of the capillary channel 1 or the like. When the length (x + y in Fig. 1A) of the capillary channel 1 is from 10 mm to 150 mm, the distance (x) from the sample holding tank 2 to the detection unit 4 is preferably from 5 mm to 140 mm, more preferably from 10 mm to 100 mm, and still more preferably from 15 mm to 50 mm.

-Alkaline Solution-

In the disclosure, "alkaline" means that the pH is more than 7.0. The pH of the alkaline solution is preferably higher than an isoelectric point of Hb, and is preferably from 7.5 to 12.0, more preferably from 8.5 to 11.0, and still more preferably from 9.5 to 10.5.

In the disclosure, the pH of the alkaline solution is the pH of the alkaline solution at 25°C, and is measured using a pH meter 30 minutes after the electrode is immersed. As the pH meter, F-72 manufactured by HORIBA, Ltd. or an apparatus similar thereto can be used.

The alkaline solution contains a cationic polymer. The alkaline solution may contain two or more types of cationic polymers.

In the disclosure, the "cationic polymer" means a polymer having a cationic group.

In the disclosure, the "cationic group" includes a cationic group and a group that is ionized to become a cationic group.

Examples of the cationic group include a primary amino group, a secondary amino group, a tertiary amino group, a quaternary ammonium base, an imino group, and the like.

The cationic polymer is preferably water-soluble. In the disclosure, "water-soluble" means that the target substance is dissolved in an amount of 1% by mass or more in water at 25°C.

Examples of the cationic polymer having a primary to tertiary amino group or a cationic group capable of being ionized into a primary to tertiary amino group include polyallylamine, polyvinylamine, polylysine, polyarginine, polyhistidine, polyornithine, polydiallylamine, polymethyldiallylamine, polyethyleneimine, diallylamine-acrylamide polymer, dimethylamine-ammonia-epichlorohydrin polymer, allylamine-diallylamine polymer, and the like. The cationic polymer described above may be in the form of a salt, and examples thereof include hydrochloride or the like.

Examples of the cationic polymer having an imino group or a cationic group capable of being ionized into an imino group include polyethyleneimine or the like.

Examples of the cationic polymer having a quaternary ammonium base or a cationic group capable of being ionized into a quaternary ammonium base include polyquaternium, a dimethylamine-ammonia-epichlorohydrin polymer, a dimethylamine-epichlorohydrin polymer, and the like.

In the disclosure, "polyquaternium" refers to a cationic polymer including a constituent unit derived from a monomer having a quaternary ammonium group. Polyquaternium can be confirmed by International Nomenclature for Cosmetic Ingredients (INCI) directory. Examples of the polyquaternium include, in one or more embodiments, polydiallyldimethylammonium salts such as polyquaternium-6(poly(diallyldimethylammoniumchloride), polyquaternium-7(co polymerofacrylamideanddiallyldimethylammoniumchloride), polyquaternium-4(diallyldimethylammoniumchloride-hy-droxyethylcellulosecopolymer), and polyquaternium-22(copolymerofacrylicacidanddiallyldimethylammoniumchloride), and polyquaternium-2 (poly[bis(2-chloroethyl)ether-alt-1,3-bis[3-(dimethylamino)propyl]urea]), and the like.

As the cationic polymer, in addition to the ammonium salt, in one or more embodiments, a cationic polymer of an onium salt such as a phosphonium salt, an oxonium salt, a sulfonium salt, a fluoronium salt, or a chloronium salt can also be used.

Examples of the cationic polymer having a hydrazide group or a cationic group capable of being ionized into a hydrazide group include aminopolyacrylamide, and the like.

From the viewpoint of shortening a separation time, a weight average molecular weight of the cationic polymer is preferably from 10,000 to 500,000.

In the disclosure, the weight average molecular weight of the cationic polymer refers to a catalogue value. When there is no catalog value of the weight average molecular weight, the weight average molecular weight is a weight average molecular weight in terms of polyethylene glycol measured by a gel permeation chromatography (GPC) method.

From the viewpoint of shortening the separation time, a content of the cationic polymer with respect to a total mass of the alkaline solution is preferably from 0.5 mass% to 10 mass%, more preferably from 0.75 mass% to 5.0 mass%, and still more preferably from 1.0 mass% to 3.0 mass%.

As the cationic polymer, one synthesized by a conventionally known method may be used, or a commercially available one may be used.

The alkaline solution may contain water. Examples of the water include distilled water, ion-exchanged water, pure water, ultrapure water, and the like.

A content of the water with respect to the total mass of the alkaline solution is not particularly limited, and may be from 10 mass% to 99.9 mass%.

The alkaline solution may contain additives such as a non-surfactant type biionic substance, a pH buffering substance, a preservative for suppressing proliferation, or the like of microorganisms, and the like. Examples of the preservative include sodium azide, ethyl paraben, procrine, and the like.

-Sample-

A sum of the content of Hb with respect to the total mass of the sample is not particularly limited, and may be from 0.001 mass% to 100 mass%.

A form of the sample is not particularly limited, and a sample may be prepared from raw material, or the raw material may be the sample itself.

Examples of the sample raw material include raw materials containing Hb, biological samples, and the like.

Examples of the biological sample include blood and a blood derivative containing a red blood cell component, and the like.

Examples of the blood include blood collected from a living body, and include blood of mammals other than human, blood of human, and the like.

Examples of the blood derivative containing a red blood cell component include those separated or prepared from blood and containing a red blood cell component. Examples thereof include a blood cell fraction from which plasma is removed, a blood cell concentrate, a lyophilizate of blood or blood cells, a hemolysis sample obtained by hemolysis of whole blood, centrifuged blood, naturally precipitated blood, washed blood cells, and the like.

From the viewpoint of improving the separation accuracy, the sample preferably contains an alkaline solution containing a cationic polymer.

The sample containing the alkaline solution can be obtained by diluting the sample raw material using the alkaline solution. A dilution rate is preferably from 1.2 times to 100 times, more preferably from 2 times to 60 times, and still more preferably from 3 times to 50 times on a mass basis. A material used for dilution is not particularly limited, and examples thereof include a pH adjusting agent (for example, hydrochloric acid or the like), a surfactant (for example, EMULGEN LS-110 (manufactured by Kao Corporation) and the like), a preservative (for example, sodium azide or the like), an ionic strength adjusting agent (for example, sodium chloride or the like), a refractive index adjusting agent (for example, saccharides such as sucrose), and the like.

The alkaline solution may be the same as or different from the alkaline solution filled in the capillary channel.

<Electropherogram Acquisition Step>

A sample analysis method includes a step of obtaining an electropherogram of a sample. Specifically, the electropherogram acquisition step preferably includes a step of optically measuring a sample to obtain an absorbance, a step of acquiring the absorbance as an original waveform plotted on a two-dimensional plane along a time axis, and a step of acquiring a time differential waveform which is a waveform obtained by differentiating the original waveform along the time axis.

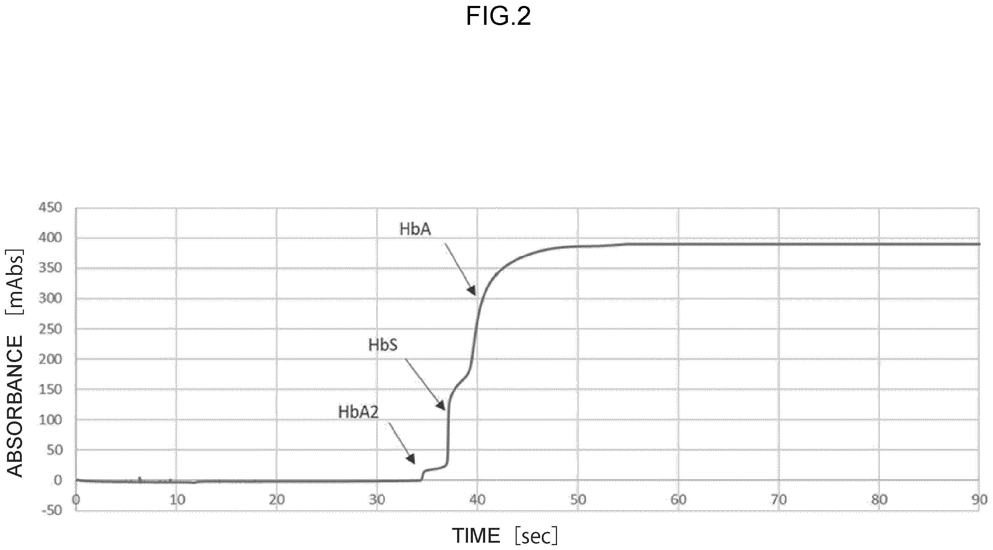

Fig. 2 is a diagram illustrating an example of the original waveform.

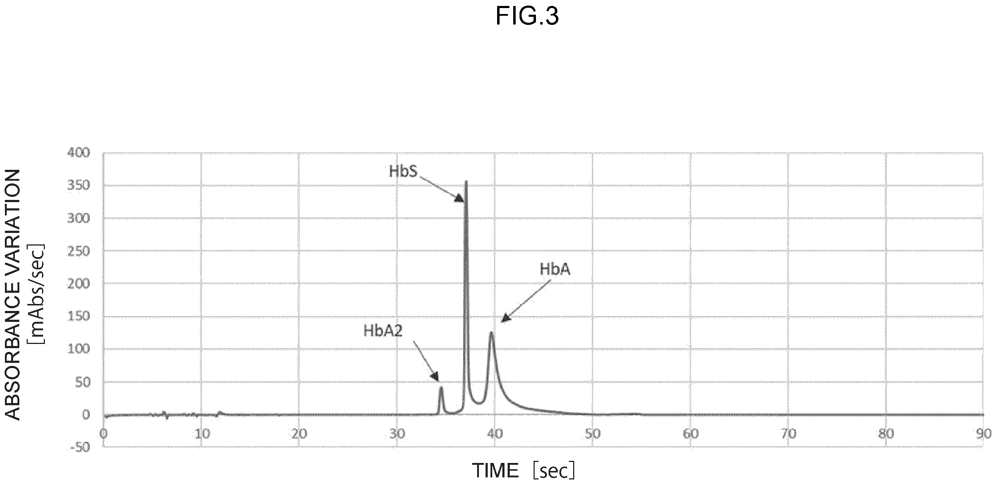

Fig. 3 is a diagram illustrating the time differential waveform with respect to the original waveform of Fig. 2.

(Step of Optically Measuring Sample to Obtain Absorbance)

The absorbance is measured, for example, by the following method.

The sample is continuously introduced into the capillary channel, and a plurality of components included in the sample in the capillary channel are separated by application of a voltage. The detection unit 4 provided in the middle of the capillary channel emits light having a wavelength of from 415 to 420 nm, and the absorbance is measured by an absorbance measuring apparatus.

For example, when the component to be analyzed is Hb, since a molecular surface thereof is negatively charged in the alkaline solution, the component to be analyzed is migrated toward an anode by disposing a cathode upstream and the anode downstream of the capillary channel.

At that time, an electrophoresis speed varies depending on a charge state of the molecular surface. That is, the stronger the negative charge on the molecular surface, the higher the electrophoresis speed. Therefore, after the sample is introduced into the capillary channel, the component having a high electrophoresis speed reaches the detection unit faster.

Further, at the time point when the component having a low electrophoresis speed reaches the detection unit, the component having a high electrophoresis speed derived from the sample introduced later also reaches the detection unit. That is, in the capillary channel, when a component having a high electrophoresis speed first reaches the detection unit, the component continues to reach the detection unit as long as the sample continues to be introduced into the capillary channel. The component having a lower electrophoresis speed reaches the detection unit with a delay, but once the component reaches the detection unit, the component also continues to reach the detection unit as long as the sample continues to be introduced into the capillary channel.

In other words, in the detection unit, a component having a high electrophoresis speed reaches first, and thereafter, a component having a lower electrophoresis speed reaches cumulatively. Therefore, the absorbance measured by the detection unit monotonically increases in a cumulative manner over time.

(Step of Acquiring Absorbance as Original Waveform Plotted on Two-Dimensional Plane Along Time Axis)

The absorbance is plotted on a two-dimensional plane having an absorbance on one axis (for example, a Y axis) and a time on the other axis (for example, an X axis) to acquire as an original waveform. Note that the absorbance may be acquired as data that can be plotted on a two-dimensional plane, and it is not essential that the absorbance is actually drawn as a graph based on such data.

(Step of Acquiring Time Differential Waveform That Is Waveform Obtained by Differentiating Original Waveform Along Time Axis)

When the original waveform is viewed along the time axis, a portion having a large gradient is derived from an increase in the absorbance due to the first reaching of a certain component at the detection unit 4. A portion having a small gradient indicates that the next component has not yet reached the detection unit 4. That is, the portion where the gradient is large in the original waveform indicates that the component in the sample solution has reached the detection unit 4. Such a portion having a large gradient in the original waveform is represented as a peak waveform appearing in a waveform (referred to as a "time differential waveform") obtained by differentiating the original waveform along the time axis. These peak waveforms are derived from each component included in the sample. In the time axis, the component corresponding to the peak waveform on a left side has a higher electrophoresis speed than the component corresponding to the peak waveform on a right side.

<Detection Time and Concentration Acquisition Step and Component Specification Step>

The sample analysis method includes a step of acquiring a detection time of each peak and a concentration corresponding to each peak from an electropherogram.

The sample analysis method includes a step of specifying a component included in the sample using a parameter indicating a correlation between a detection time and a concentration calculated using a known component with respect to the acquired detection time and the acquired concentration.

The detection time and concentration acquisition step preferably includes a step of acquiring a detection time of each peak in a time differential waveform and acquiring an absorbance corresponding to the acquired detection time of each peak.

The component specification step preferably includes a step of specifying a component included in the sample using a parameter indicating a correlation between a detection time and an absorbance calculated using a known component with respect to the acquired detection time and the acquired absorbance.

The parameter (hereinafter, also referred to as "specific parameter") indicating the correlation between the detection time and the absorbance calculated using the known component is preferably set in advance in the sample analysis apparatus before the sample to be analyzed is analyzed.

The specific parameter is preferably a parameter obtained by plotting the detection time and the absorbance on the two-dimensional plane using the known component.

Hereinafter, a specific method of specifying a component included in a sample using a specific parameter will be described.

(Method A)

In a method A, a specific parameter is data of a slope of an approximate straight line for each component, obtained by plotting a detection time and an absorbance on a two-dimensional plane using a known component.

In the method A, the step of specifying the component included in the sample includes a step of correcting the acquired detection time using the data of the slope and calculating a correction detection time, and a step of specifying a component included in the sample based on the correction detection time.

The specific parameter in the method A is calculated, for example, by the following method.

By capillary electrophoresis, the absorbance is obtained using known Hb species with different concentration. The absorbance is acquired as an original waveform A plotted on a two-dimensional plane along a time axis, and a time differential waveform A that is a waveform obtained by differentiating the original waveform A along the time axis is acquired. The X axis represents a time, and the Y axis represents an absorbance variation.

The following data is acquired from the time differential waveform A.

·PkTime: means a detection time at PkTop where a maximum value of the peak is PkTop.
·PkBtTime: means a detection time at PkBt where a minimum value immediately before PkTop is PkBt.
·PkTlTime: means a detection time at PkTl where a minimum value immediately after PkTop is PkTl.
·EOFTime: means an earliest detection time among the detection times at PkTop where the absorbance variation exceeds 0.2 mAbs/sec.
·PkArea: means an integrated value of the absorbance variation from PkBtTime to PkTlTime in PkTop.
·Pk%: means a proportion (%) of each PkArea in all PkAreas.
·A2Time: means PkTime of HbA2. PkTime at the peak at which Pk% was 1% or more immediately after EOFTime was A2Time.

The PkTime is converted into a relative detection time PkTimeRt based on the following Formula A.

$$\mathrm{PkTimeRt = (PkTime - EOFTime)/(A2Time - EOFTime)... \ \ Formula \ A}$$

In the above Formula A, the relative detection time is calculated using A2Time, but instead of A2Time, the detection time of HbA in the blood or a protein added to the sample from the outside (for example, protein absorbing a wavelength of 415 nm and indicating the same behavior as Hb) may be used.

Next, an absorbance TlAbs corresponding to PkTlTime is acquired from the original waveform A.

Instead of the absorbance TlAbs, an absorbance corresponding to PkTime may be acquired.

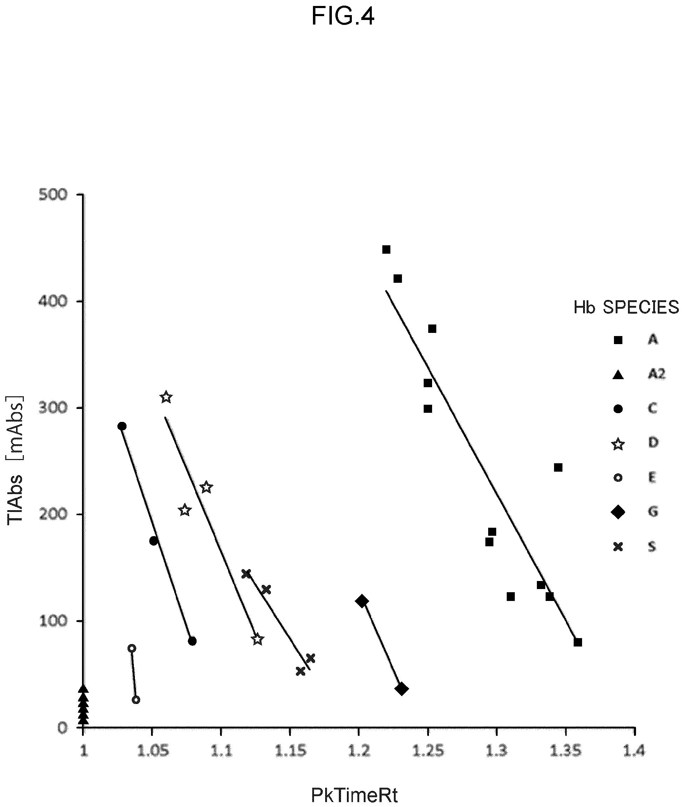

Fig. 4 is a diagram in which a horizontal axis is a relative detection time PkTimeRt and a vertical axis is an absorbance TlAbs.

As illustrated in Fig. 4, an approximate straight line is drawn for each Hb species, and each slope of the approximate straight line is calculated. Further, an average value of the slopes of the approximate straight lines is calculated.

The correction detection time is calculated for each Hb species based on the following Formula B.

Correction detection rate = PkTimeRt + {T1Abs / (- average value of slopes of approximate straight lines)}    formula b

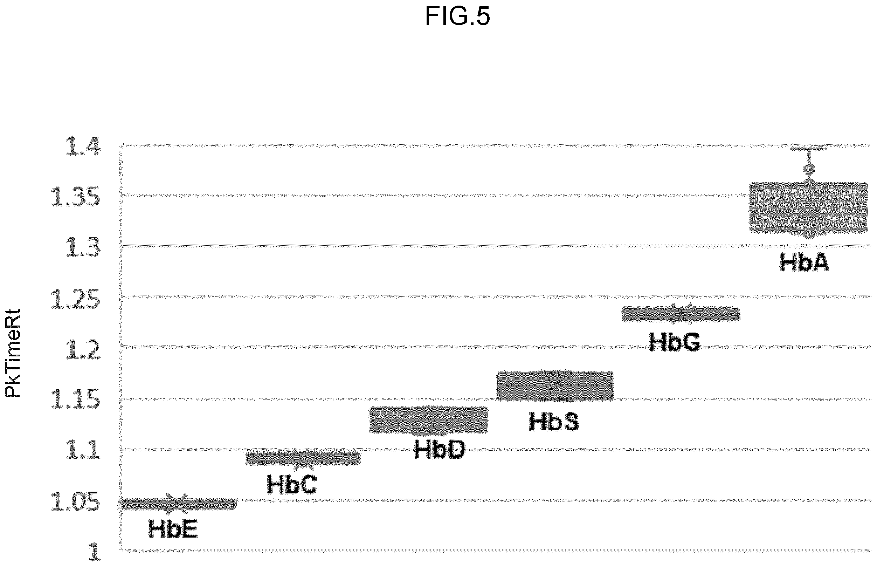

Fig. 5 is a diagram illustrating an example of a relationship between Hb species and a correction detection time.

From Fig. 5, the Hb species and the range of the correction detection time corresponding to the Hb species are determined.

·HbE: The correction detection time is 1.010 or more and less than 1.070.
·HbC: The correction detection time is 1.070 or more and less than 1.110.
·HbD: The correction detection time is 1.110 or more and less than 1.145.
·HbS: The correction detection time is 1.145 or more and less than 1.190.
·HbG: The correction detection time is 1.190 or more and less than 1.250.
·HbA: The correction detection time is 1.250 or more and less than 1.450.

As illustrated in Fig. 5, the correction detection times do not overlap depending on the Hb species. Therefore, it is possible to acquire the relative detection time PkTimeRt and the absorbance TlAbs for the sample to be analyzed, calculate the correction detection time based on Formula B, and specify Hb species included in the sample based on the correction detection time.

The method of acquiring the relative detection time PkTimeRt and the absorbance TlAbs for the sample to be analyzed (sample in which the Hb species included is unknown) is similar to the method of acquiring the relative detection time PkTimeRt and the absorbance TlAbs for known Hb species having different concentration.

(Method B)

In a method B, the specific parameter is data of a boundary line separating each component, obtained by plotting a detection time and an absorbance on a two-dimensional plane using a known component.

In the method B, the step of specifying the component included in the sample includes a step of specifying a region to which the acquired detection time and the acquired absorbance belong, in each region separated by the boundary line using the data of the boundary line, and a step of specifying a component included in the sample based on the specified region.

The specific parameter in the method B is calculated, for example, by the following method.

Similarly to the method A, the relative detection time PkTimeRt and the absorbance TlAbs are acquired for known Hb species with different concentration.

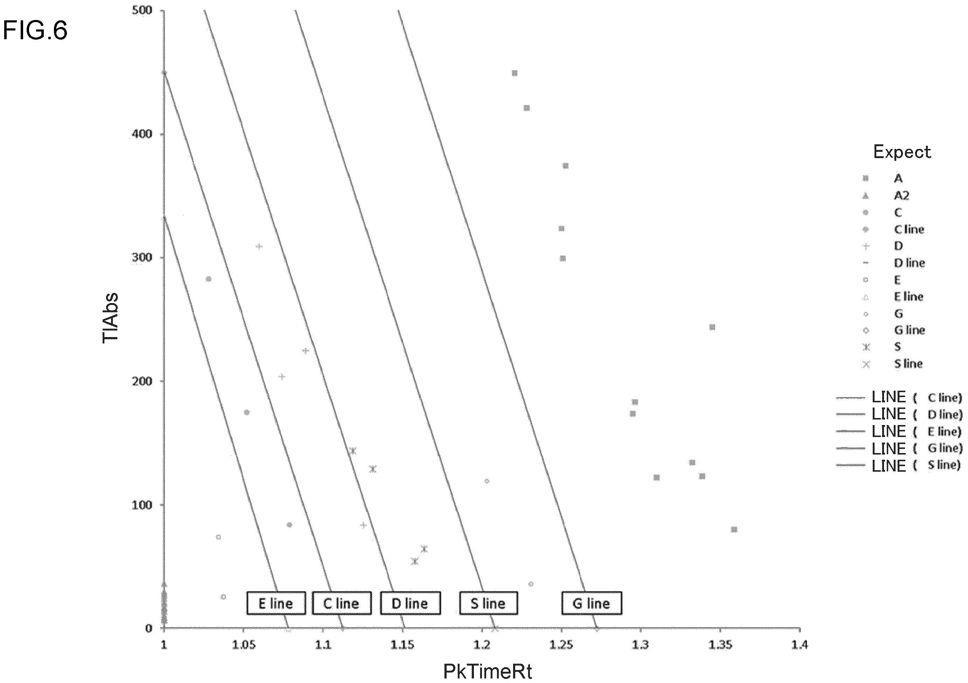

Fig. 6 is a diagram in which a horizontal axis represents the relative detection time PkTimeRt and a vertical axis represents the absorbance TlAbs.

As illustrated in Fig. 6, boundary lines to be separated for each Hb species are drawn, and linear expressions of the boundary lines are calculated.

E line is a boundary line separating HbE and HbC.
C line is a boundary line separating HbC and HbD.
D line is a boundary line separating HbD and HbS.
S line is a boundary line separating HbS and HbG.
G line is a boundary line separating HbG and HbA.

The linear expression of each boundary line is set as follows, for example.

E line: TlAbs = 4583 - 4250 $\times$ PkTimeRt
C line: TlAbs = 4450 - 4000 $\times$ PkTimeRt
D line: TlAbs = 4550 - 3950 $\times$ PkTimeRt
S line: TlAbs = 4794 - 3967 $\times$ PkTimeRt
G line: TlAbs = 5079 - 3991 $\times$ PkTimeRt

As illustrated in Fig. 6, the Hb species are separated by the boundary lines. Therefore, the relative detection time PkTimeRt and the absorbance TlAbs are acquired for the sample to be analyzed, and in each region separated by the boundary line, a region to which the acquired relative detection time and the acquired absorbance belong is specified. Based on the specified region, Hb species included in the sample can be specified.

When the following Formulas E1 and E2 are satisfied, it is specified as HbE.

$$PkTimeRt > 1... \text{ Formula E1}$$

$$TlAbs < 4583 - 4250 \times PkTimeRt... \text{ Formula E2}$$

When the following Formula C1 is satisfied, it is specified as HbC.

$$4583 - 4250 \times PkTimeRt \leq TlAbs < 4450 - 4000 \times PkTimeRt... \text{ Formula C1}$$

When the following Formula D1 is satisfied, it is specified as HbD.

$$4450 - 4000 \times PkTimeRt \leq TlAbs < 4550 - 3950 \times PkTimeRt... \text{ Formula D1}$$

When the following Formula S1 is satisfied, it is specified as HbS.

$$4550 - 3950 \times \text{PkTimeRt} \leq \text{TlAbs} < 4794 - 3967 \times \text{PkTimeRt}... \text{ Formula S1}$$

When the following Formula G1 is satisfied, it is specified as HbG.

$$4794 - 3967 \times \text{PkTimeRt} \leq \text{TlAbs} < 5079 - 3991 \times \text{PkTimeRt}... \text{ Formula G1}$$

When the following Formula A1 is satisfied, it is specified as HbA.

$$5079 - 3991 \times \text{PkTimeRt} \leq \text{TlAbs}... \text{ Formula A1}$$

[Sample Analysis Apparatus]

The sample analysis apparatus includes means for separating a plurality of components included in a sample by capillary electrophoresis, means for obtaining an electropherogram of the sample, means for acquiring a detection time of each peak and a concentration corresponding to each peak from the electropherogram, and means for specifying a component included in the sample using a parameter indicating a correlation between a detection time and a concentration calculated using a known component with respect to the acquired detection time and the acquired concentration.

The means for obtaining the electropherogram of the sample preferably includes means for optically measuring the sample to obtain an absorbance, means for acquiring the absorbance as an original waveform plotted on a two-dimensional plane along a time axis, and means for acquiring a time differential waveform which is a waveform obtained by differentiating the original waveform along the time axis.

The means for acquiring the detection time of each peak and the concentration corresponding to each peak from the electropherogram preferably includes means for acquiring the detection time of each peak in the time differential waveform and acquiring the absorbance corresponding to the acquired detection time of each peak.

The means for specifying the component included in the sample preferably includes a step of specifying a component included in the sample using a parameter indicating a correlation between a detection time and an absorbance calculated using a known component with respect to the acquired detection time and the acquired absorbance.

Specifically, the parameter indicating the correlation between the detection time and the concentration is data of a slope of an approximate straight line for each component, obtained by plotting the detection time and the absorbance on a two-dimensional plane using a known component, and the step of specifying the component included in the sample preferably includes means for correcting the acquired detection time using the data of the slope and calculating the correction detection time, and means for specifying a component included in the sample based on the correction detection time.

The parameter indicating the correlation between the detection time and the concentration is data of a boundary line separating each component, obtained by plotting the detection time and the absorbance on a two-dimensional plane using a known component, and the means for specifying the component included in the sample preferably includes means for specifying a region to which the acquired detection time and the acquired absorbance belong, in each region separated by the boundary line using the data of the boundary line, and means for specifying a component included in the sample based on the specified region.

<Sample Analysis System>

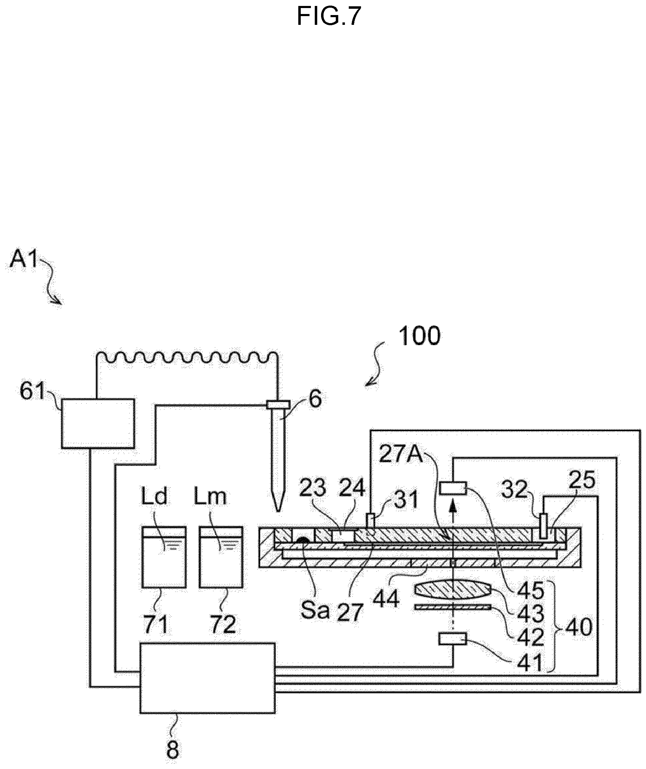

Fig. 7 illustrates a schematic configuration of a sample analysis system A1 that can be used to perform the sample analysis method according to the present embodiment. The sample analysis system A1 includes a sample analysis apparatus 100 and an analysis chip 10. The sample analysis system A1 is a system that executes an analysis method by the capillary electrophoresis method on a sample Sa.

The analysis chip 10 holds the sample Sa and provides a field of analysis for the sample Sa in a state of being loaded in the sample analysis apparatus 100. As illustrated in Figs. 8 and 9, the analysis chip 10 includes a main body 21, a mixing tank 22, an introduction tank 23, a filter 24, a discharge tank 25, an electrode tank 26, a channel 27, and a communication channel 28. Fig. 8 is a plan diagram of the analysis chip 10, and Fig. 9 is a cross-sectional diagram taken along line III-III of Fig. 8. The analysis chip 10 is not limited to a disposable type, and may be used for a plurality of times of analysis. The sample analysis system of the present embodiment is not limited to the configuration including the separate analysis chip 10 loaded in the sample analysis apparatus 100, and may have a configuration in which a functional site having a function similar to that of the analysis chip 10 is incorporated in the sample analysis apparatus 100.

The main body 21 serves as a base of the analysis chip 10. A material of the main body 21 is as described above for the material of the capillary channel. In the present embodiment, the main body 21 has a configuration in which an upper portion 2A and a lower portion 2B in Fig. 9 are formed separately and coupled to each other. The invention is not limited thereto, and for example, the main body 21 may be integrally formed.

The mixing tank 22 is a portion where the sample Sa and a diluent Ld are mixed. The mixing tank 22 is configured as a recess opened upward by, for example, a through-hole formed in the upper portion 2A of the main body 21. The introduction tank 23 is a tank into which a sample solution obtained by mixing the sample Sa and the diluent Ld in the mixing tank 22 is introduced. The introduction tank 23 is configured as a recess opened upward by, for example, the through-hole formed in the upper portion 2A of the main body 21.

The filter 24 is provided at an opening of the introduction tank 23, which is an example of an introduction path into the introduction tank 23. The specific configuration of the filter 24 is not limited, and suitable examples thereof include a cellulose acetate membrane filter (manufactured by ADVANTEC Corporation, pore diameter 0.45 μm).

The discharge tank 25 is a tank located on a downstream side of the channel 27. For example, the discharge tank 25 is configured as a recess opened upward by the through-hole formed in the upper portion 2A of the main body 21. The electrode tank 26 is a tank into which an electrode 31 is inserted in the capillary electrophoresis method.

The electrode tank 26 is configured as a recess opened upward by, for example, the through-hole formed in the upper portion 2A of the main body 21. The communication channel 28 connects the introduction tank 23 and the electrode tank 26, and forms a conduction path between the introduction tank 23 and the electrode tank 26.

The channel 27 is formed as a capillary tube connecting the introduction tank 23 and the discharge tank 25, and is a field where an electro-osmotic flow (EOF) in an electrophoresis method is generated. The channel 27 is configured as, for example, a groove formed in the lower portion 2B of the main body 21. In the main body 21, a recess or the like for promoting irradiation of the channel 27 with light and emission of light transmitted through the channel 27 may be appropriately formed. A cross-sectional shape and size (channel height and channel width in the case of rectangular shape, and inner diameter and channel length in the case of circular shape) of the channel 27 are as described in the cross-sectional shape and size of the capillary channel. The size of the entire analysis chip 10 is appropriately set according to the size of the channel 27 and the size, arrangement, and the like of the mixing tank 22, the introduction tank 23, the discharge tank 25, and the electrode tank 26.

The analysis chip 10 having the above configuration is an example, and an analysis chip having a configuration capable of component analysis by a capillary electrophoresis method can be appropriately adopted.

The sample analysis apparatus 100 performs analysis processing on the sample Sa in a state where the analysis chip 10 on which the sample Sa is spotted is loaded. The sample analysis apparatus 100 includes an electrode 31, an electrode 32, a light source 41, an optical filter 42, a lens 43, a slit 44, a detector 45, a dispenser 6, a pump 61, a diluent tank 71, an electrophoresis liquid tank 72, and a control unit 8. The light source 41, the optical filter 42, the lens 43, and the detector 45 configure a measurement unit 40 in the present embodiment.

The electrode 31 and the electrode 32 are for applying a predetermined voltage to the channel 27 in the capillary electrophoresis method. The electrode 31 is inserted into the electrode tank 26 of the analysis chip 10, and the electrode 32 is inserted into the discharge tank 25 of the analysis chip 10. The voltage applied to the electrode 31 and the electrode 32 is as described above with reference to the voltage applied to both ends of the capillary channel.

The light source 41 is a portion that emits light for measuring an absorbance as an optical measurement value in the capillary electrophoresis method. The light source 41 includes, for example, an LED chip that emits light in a predetermined wavelength region. The optical filter 42 attenuates light having a predetermined wavelength among the light from the light source 41 and transmits light having a remaining wavelength. The lens 43 is for condensing the light transmitted through the optical filter 42 to the analysis portion of the channel 27 of the analysis chip 10. The slit 44 is for removing extra light that may cause scattering or the like among the light condensed by the lens 43.

The detector 45 receives light from the light source 41 that has transmitted through the channel 27 of the analysis chip 10, and includes, for example, a photodiode, a photo IC, or the like.

As described above, a path through which the light emitted from the light source 41 reaches the detector 45 is an optical path. Further, an optical measurement value of a solution (that is, any one of the sample solution and the electrophoresis liquid or a mixed solution thereof) flowing through the channel 27 is measured at a measurement position 27A where the optical path intersects with the channel 27. That is, the measurement unit 40 measures the optical measurement value of the sample solution at the measurement position 27A in the channel 27. Examples of the optical measurement value include an absorbance. The absorbance represents a degree to which the light in the optical path is absorbed by the solution flowing through the channel 27, and represents an absolute value of a common logarithm value of a ratio between an incident light intensity and a transmitted light intensity. In this case, a general-purpose spectrophotometer can be used as the detector 45. Without using the absorbance, any optical measurement value such as the value itself of the transmitted light intensity can be used. Hereinafter, a case in which the absorbance is used as the optical measurement value will be described as an example.

The dispenser 6 dispenses a desired amount of a diluent Ld, an electrophoresis liquid Lm, and a sample solution, and includes, for example, a nozzle. The dispenser 6 is freely movable at a plurality of predetermined positions in the sample analysis apparatus 100 by a drive mechanism (not illustrated). The pump 61 is a suction source and a discharge source to the dispenser 6. The pump 61 may be used as a suction source and a discharge source of a port (not illustrated) provided in the sample analysis apparatus 100. These ports are used for filling the electrophoresis liquid Lm, and the like. A dedicated pump different from the pump 61 may be provided.

The diluent tank 71 is a tank for storing the diluent Ld. The diluent tank 71 may be a tank permanently installed in the sample analysis apparatus 100, or a container in which a predetermined amount of the diluent Ld is sealed may be loaded in the sample analysis apparatus 100. The electrophoresis liquid tank 72 is a tank for storing the electrophoresis liquid Lm. The electrophoresis liquid tank 72 may be a tank permanently installed in the sample analysis apparatus 100, or a container in which a predetermined amount of the electrophoresis liquid Lm is sealed may be loaded in the sample analysis apparatus 100.

The diluent Ld is mixed with the sample Sa to generate a sample solution. A main agent of the diluent Ld is not particularly limited, and examples thereof include water and physiological saline, and preferred examples thereof include a liquid having a component similar to that of the electrophoresis liquid Lm described later. In addition to the main agent, an additive may be added to the diluent Ld as necessary.

The electrophoresis liquid Lm is a medium that is filled in the discharge tank 25 and the channel 27 in the analysis step by the electrophoresis method and generates an electro-osmotic flow in the electrophoresis method. The preferred aspect of the electrophoresis liquid Lm is as described above. An additive may be added to the electrophoresis liquid Lm as necessary, similarly to the description of the diluent Ld.

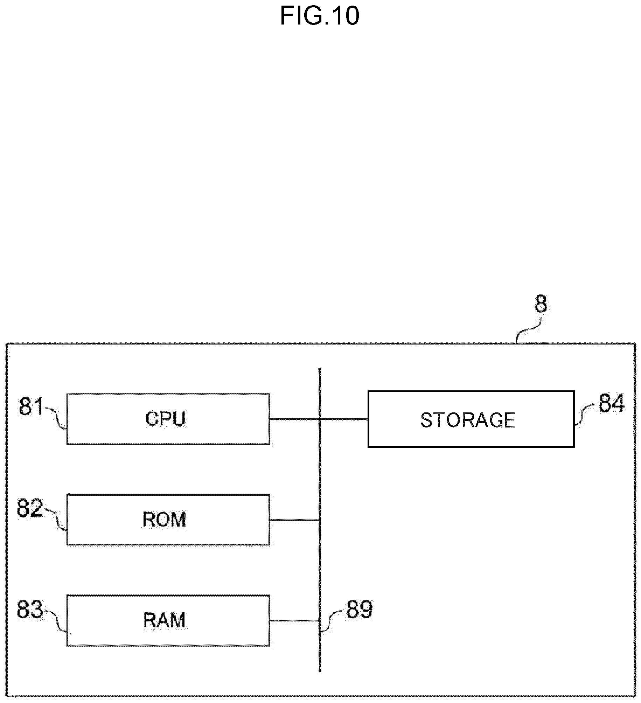

The control unit 8 controls each unit in the sample analysis apparatus 100. As illustrated in the hardware configuration of Fig. 10, the control unit 8 has a central processing unit (CPU) 81, a read only memory (ROM) 82, a random access memory (RAM) 83, and a storage 84.

The respective components are communicably coupled to each other via a bus 89.

The CPU 81 is a central processing unit, and executes various programs and controls each unit. That is, the CPU 81 reads a program from the ROM 82 or the storage 84, and executes the program using the RAM 83 as a work area. The CPU 81 performs control of each of the above-described configurations and various types of arithmetic processing according to the program recorded in the ROM 82 or the storage 84.

The ROM 82 stores various programs and various data. The RAM 83 temporarily stores programs or data as a work area. The storage 84 includes a hard disk drive (HDD), a solid state drive (SSD), or a flash memory, and stores various programs including an operating system and various data.

In the present embodiment, the ROM 82 or the storage 84 stores programs and various data for executing the sample analysis method according to the present embodiment.

When executing the sample analysis method according to the present embodiment, the sample analysis apparatus 100 performs measurement and analysis using the above hardware resources and the above configurations.

Examples

Examples will be described below, but the disclosure is not limited to these examples at all. In the following description, unless otherwise specified, "part" and "%" are all on a mass basis.

<Separation Device and Measuring Apparatus>

As the separation device, a resin-made chip (channel width: 40 $\mu$m, channel height: 40 $\mu$m, channel length: 30 mm, and distance (x) from sample holding tank 2 to detection unit 4: 20 mm) having the capillary channel 1 having the structure illustrated in Fig. 1 was used. A capacity of the sample holding tank 2 and the electrophoresis liquid holding tank 3 was 10 $\mu$L. The inner wall of the capillary channel was coated with polydiallyldimethylammonium chloride.

As a separation device, The Lab 001A1c HD (ARKRAY,Inc.) was used, and as a measurement apparatus, The Lab 001 (ARKRAY,Inc.) was used.

<Preparation of Capillary Electrophoresis Solution>

The following substances were mixed, and sodium hydroxide and water were added until the pH reached 9.8 to prepare a capillary electrophoresis solution.

·Dimethylamine-ammonia-epichlorohydrin polycondensate (cationic polymer, Unisense KHE 1000 L, weight average molecular weight from 100,000 to 500,000, manufactured by Senka Corporation)... 1.5 mass%
·Sodium azide... 0.02 mass%
·Sodium hydroxide
·Water

<Preparation of Diluent Solution>

The following substances were mixed, and hydrochloric acid and water were added until the pH reached 8.8 to prepare a capillary diluent solution.

·Polyethyleneimine (manufactured by FUJIFILM Wako Pure Chemical Corporation, weight average molecular weight 70,000)... 1 mass%
·EMULGEN LS-110 (manufactured by Kao Corporation)... 0.4 mass%
·Sodium azide... 0.02 mass%
·Sucrose... 7.87 mass%
·Sodium chloride... 0.26 mass%
·Hydrochloric acid
·Water

<Preparation of Hb Specimen>

HbA2 is included in both whole blood of a healthy subject and whole blood of various mutant Hb carriers.

·Whole blood of a healthy subject: AA (HbA/HbA)
·Whole blood of various mutant Hb carriers: AE (HbA/HbE), AC (HbA/HbC), AD (HbA/HbD), AS (HbA/HbS), AG (HbA/HbG), CC (HbC/HbC), DD (HbD/HbD), and SS

(Hb S/Hb S)

[Example 1]

(Setting of Specific Parameter)

-Separation Step-

9 μL of the capillary electrophoresis solution was supplied to the electrophoresis liquid holding tank 3 in the capillary electrophoresis chip, and the capillary channel 1 was filled with the capillary electrophoresis solution by capillary phenomenon.
To the sample holding tank 2, 9 μL of a sample obtained by diluting the whole blood of the healthy subject and the whole blood of various mutant Hb carriers with a diluent solution at a dilution ratio of 41 times or 121 times was supplied.
Next, a negative electrode was brought into contact with the sample holding tank 2, and a positive electrode was brought into contact with the electrophoresis liquid holding tank 3, and electrophoresis was started by applying a voltage under constant current control of 75 μA.
The electrophoresis was performed for 90 seconds.

-Electropherogram Acquisition Step-

During the electrophoresis, the detection unit 4 was irradiated with light of 415 nm, and an absorbance was measured to obtain an original waveform A of an absorbance spectrum.
The original waveform A was differentiated along the time axis to obtain the time differential waveform A.
The Lab 001 manufactured by ARKRAY,Inc. was used for the light irradiation, measurement of the absorbance, and acquisition of the electropherogram.
For the whole blood of the healthy subject and the whole blood of the various mutant Hb carriers, the above measurement was performed to acquire the electropherogram. The capillary electrophoresis chip was disposable for each measurement.

-Detection Time and Concentration Acquisition Step-

PkTime, EOFTime, PkTITime, and A2Time were acquired from the time differential waveform A.
Based on the following Formula A, PkTime was converted into the relative detection time PkTimeRt.

$$PkTimeRt = (PkTime - EOFTime)/(A2Time - EOFTime)...\ Formula\ A$$

The absorbance TIAbs corresponding to PkTITime was acquired from the original waveform A.

In Fig. 4 in which a horizontal axis represents the relative detection time PkTimeRt and a vertical axis represents the absorbance TIAbs, an approximate straight line was drawn for each Hb species, and slopes of the approximate straight lines were calculated. The average value of the slopes of the approximate straight lines was -4923.

The correction detection time was calculated based on the following Formula B.

Correction detention time = PkTimeRt + {T1Abs / (- average value of slopes of approximate straight lines)}        formula b

From Fig. 5, the Hb species and the range of the correction detection time corresponding to the Hb species were determined.

·HbE: The correction detection time is 1.010 or more and less than 1.070.
·HbC: The correction detection time is 1.070 or more and less than 1.110.
·HbD: The correction detection time is 1.110 or more and less than 1.145.
·HbS: The correction detection time is 1.145 or more and less than 1.190.
·HbG: The correction detection time is 1.190 or more and less than 1.250.
·HbA: The correction detection time is 1.250 or more and less than 1.450.

(Component Specification Step)

It was determined which one of the above ranges of the correction detection time the obtained correction detection time corresponds to, and the Hb species could be specified.

[Example 2]

In the sample holding tank 2, components were separated in the same manner as in the case of using the whole blood of the healthy subject and the whole blood of the various mutant Hb carriers, and an electropherogram was acquired.

The relative detection time PkTimeRt and the absorbance TIAbs were acquired from the electropherogram.

Fig. 6 is a diagram in which a horizontal axis represents the relative detection time PkTimeRt and a vertical axis represents the absorbance TIAbs.

As illustrated in Fig. 6, boundary lines to be separated for each Hb species were drawn, and linear expressions of the boundary lines were calculated.

The linear expression of each boundary line was set as follows.

E line: TIAbs = 4583 - 4250 × PkTimeRt

C line: TIAbs = 4450 - 4000 × PkTimeRt

D line: TIAbs = 4550 - 3950 × PkTimeRt

S line: TIAbs = 4794 - 3967 × PkTimeRt

G line: TIAbs = 5079 - 3991 × PkTimeRt

(Component Specification Step)

A region to which the obtained relative detection time PkTimeRt and the absorbance TIAbs belong was specified, and Hb species could be specified based on the specified region.

[Comparative Example 1]

As in Example 1, the relative detection time PkTimeRt was acquired using the whole blood of the healthy subject and the whole blood of the various mutant Hb carriers.

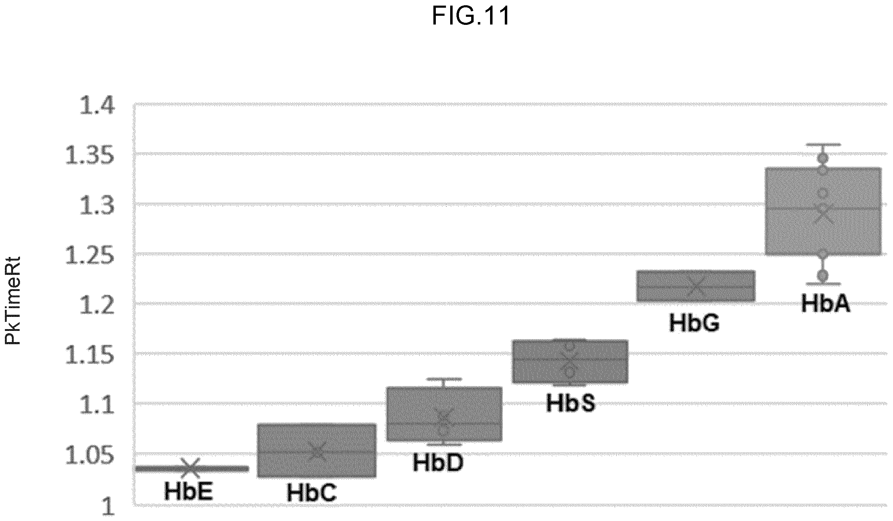

Fig. 11 is a diagram illustrating a relationship between the Hb species and the relative detection time.

As illustrated in Fig. 11, there is a portion where the relative detection times PkTimeRt overlap with each Hb species. That is, it can be seen that the components included in the sample cannot be specified only by the relative detection time PkTimeRt.

Description of Reference Numerals

| | |
|---|---|
| 1 | Capillary channel |
| 2 | Sample holding tank |
| 3 | Electrophoresis liquid holding tank |
| 4 | Detection unit |
| Tx | Detection time of X peak |
| Ta2 | Detection time of HbA2 peak |
| Ty | Detection time of Y peak |
| A1 | Measurement system |
| Sa | Sample |
| Ld | Diluent |
| Lm | Electrophoresis liquid |
| 100 | Component analysis apparatus |
| 10 | Analysis chip |
| 2A | Upper portion |
| 2B | Lower portion |
| 21 | Main body |
| 22 | Mixing tank |
| 23 | Introduction tank |
| 24 | Filter |
| 25 | Discharge tank |
| 26 | Electrode tank |
| 27 | Channel |
| 27A | Measurement position |
| 28 | Communication channel |
| 31 | Electrode |
| 32 | Electrode |
| 40 | Measurement unit |
| 41 | Light source |
| 42 | Optical filter |
| 43 | Lens |
| 44 | Slit |
| 45 | Detector |
| 6 | Dispenser |
| 61 | Pump |
| 71 | Diluent tank |
| 72 | Electrophoresis liquid tank |
| 8 | Control unit |
| 81 | CPU |
| 82 | ROM |
| 83 | RAM |
| 84 | Storage |
| 89 | Bus |

**Claims**

1. A sample analysis method comprising:

   a step of separating a plurality of components included in a sample by capillary electrophoresis;
   a step of obtaining an electropherogram of the sample;
   a step of acquiring a detection time of each peak and a concentration corresponding to each peak from the electropherogram; and
   a step of specifying a component included in the sample using a parameter indicating a correlation between a detection time and a concentration calculated using a known component with respect to the acquired detection time and the acquired concentration.

2. The sample analysis method according to claim 1, wherein:

the step of obtaining the electropherogram of the sample includes:

a step of optically measuring the sample to obtain an absorbance,
a step of acquiring the absorbance as an original waveform plotted on a two-dimensional plane along a time axis, and
a step of acquiring a time differential waveform that is a waveform obtained by differentiating the original waveform along the time axis;

the step of acquiring the detection time of each peak and the concentration corresponding to each peak from the electropherogram includes:
a step of acquiring a detection time of each peak in the time differential waveform and acquiring an absorbance corresponding to the acquired detection time of each peak; and
the step of specifying the component included in the sample includes:
a step of specifying a component included in the sample using a parameter indicating a correlation between a detection time and an absorbance calculated using a known component with respect to the acquired detection time and the acquired absorbance.

3. The sample analysis method according to claim 1 or 2, wherein the parameter indicating the correlation between the detection time and the concentration is a parameter obtained by plotting the detection time and the absorbance on the two-dimensional plane using the known component.

4. The sample analysis method according to claim 3, wherein:

the parameter indicating the correlation between the detection time and the concentration is data of a slope of an approximate straight line for each component, obtained by plotting the detection time and the absorbance on the two-dimensional plane using the known component; and
the step of specifying the component included in the sample includes:

a step of correcting the acquired detection time using the data of the slope and calculating a correction detection time, and
a step of specifying a component included in the sample based on the correction detection time.

5. The sample analysis method according to claim 3, wherein:

the parameter indicating the correlation between the detection time and the concentration is data of a boundary line separating each component, obtained by plotting the detection time and the absorbance on the two-dimensional plane using the known component; and
the step of specifying the component included in the sample includes:

a step of specifying a region to which the acquired detection time and the acquired absorbance belong, in each region separated by the boundary line using the data of the boundary line, and
a step of specifying a component included in the sample based on the specified region.

6. The sample analysis method according to any preceding claim, wherein the component is hemoglobin.

7. A sample analysis apparatus comprising:

means for separating a plurality of components included in a sample by capillary electrophoresis;
means for obtaining an electropherogram of the sample;
means for acquiring a detection time of each peak and a concentration corresponding to each peak from the electropherogram; and
means for specifying a component included in the sample using a parameter indicating a correlation between a detection time and a concentration calculated using a known component with respect to the acquired detection time and the acquired concentration.

# FIG.1

FIG.2

# FIG.3

EP 4 481 376 A1

# FIG.4

FIG.5

FIG.6

# FIG.7

A1

100

61

6

Ld    Lm

23  24    27A

31            32    25

71    72

Sa  27    44    45
                43
                42
            41    40

8

FIG.8

EP 4 481 376 A1

FIG.9

# FIG.10

FIG.11

Europäisches
Patentamt

European
Patent Office

Office européen
des brevets

# EUROPEAN SEARCH REPORT

**Application Number**

EP 24 18 2272

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHIHABI Z K ED - CHANKVETADZE B ET AL: "Effect of sample composition on electrophoretic migration - Application to hemoglobin analysis by capillary electrophoresis and agarose electrophoresis", JOURNAL OF CHROMATOGRAPHY A, ELSEVIER, AMSTERDAM, NL, vol. 1027, no. 1-2, 20 February 2004 (2004-02-20), pages 179-184, XP004483916, ISSN: 0021-9673, DOI: 10.1016/J.CHROMA.2003.08.054 | 1-3,6,7 | INV. G01N27/447 |
| Y | * abstract * | 2,3 | |
| A | * page 179, left-hand column, paragraph 1 - page 180, right-hand column, paragraph 3 * <br> * page 182, right-hand column, paragraph 1 - page 183, right-hand column, last paragraph; figures 3-5 * | 4,5 | |
| Y | EP 3 754 330 A1 (ARKRAY INC [JP]) 23 December 2020 (2020-12-23) | 2,3 | TECHNICAL FIELDS SEARCHED (IPC) |
| A | * abstract * <br> * paragraphs [0071] - [0075]; figure 14 * | 4,5 | G01N |
| A | US 2019/120790 A1 (KAWANO MASAO [JP] ET AL) 25 April 2019 (2019-04-25) <br> * abstract * <br> * paragraphs [0094] - [0097], [0102] - [0103]; figure 12 * | 1-7 | |
| A | US 2020/072790 A1 (YOSHIDA JIN [JP]) 5 March 2020 (2020-03-05) <br> * abstract * <br> * paragraph [0057] * | 1-7 | |

The present search report has been drawn up for all claims

| Place of search | Date of completion of the search | Examiner |
|---|---|---|
| Munich | 11 November 2024 | Meltaus, Johanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 2272

This annex lists the patent family members relating to the patent documents cited in the above-mentioned European search report.
The members are as contained in the European Patent Office EDP file on
The European Patent Office is in no way liable for these particulars which are merely given for the purpose of information.

11-11-2024

| Patent document cited in search report | | Publication date | Patent family member(s) | | Publication date |
|---|---|---|---|---|---|
| EP 3754330 | A1 | 23-12-2020 | CN | 112114021 A | 22-12-2020 |
| | | | EP | 3754330 A1 | 23-12-2020 |
| US 2019120790 | A1 | 25-04-2019 | CN | 109696464 A | 30-04-2019 |
| | | | EP | 3477292 A1 | 01-05-2019 |
| | | | JP | 6871129 B2 | 12-05-2021 |
| | | | JP | 2019078599 A | 23-05-2019 |
| | | | US | 2019120790 A1 | 25-04-2019 |
| US 2020072790 | A1 | 05-03-2020 | CN | 110857929 A | 03-03-2020 |
| | | | EP | 3614134 A1 | 26-02-2020 |
| | | | JP | 7085943 B2 | 17-06-2022 |
| | | | JP | 2020030116 A | 27-02-2020 |
| | | | US | 2020072790 A1 | 05-03-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016136135 A
- JP 6846324 B

- JP 6871129 B